(19) Europäisches Patentamt
European Patent Office
Office européen des brevets

(11) **EP 3 009 138 B1**

(12) **EUROPEAN PATENT SPECIFICATION**

(45) Date of publication and mention of the grant of the patent:
**01.05.2019 Bulletin 2019/18**

(51) Int Cl.:
*A61K 36/22* (2006.01)　　*A23L 33/00* (2016.01)
*A23L 33/105* (2016.01)　　*A61P 3/04* (2006.01)

(21) Application number: **15183167.4**

(22) Date of filing: **31.08.2015**

(54) **MANGIFERA EXTRACT FOR THE MANAGEMENT OF OBESITY AND/OR WEIGHT**

MANGIFERA EXTRAKT ZUR KONTROLLE VON ADIPOSITAS UND/ODER GEWICHT

EXTRAIT DE MANGIFERA POUR LA GESTION DE L'OBÉSITÉ ET/OU DU POIDS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2014 US 201462044398 P**

(43) Date of publication of application:
**20.04.2016 Bulletin 2016/16**

(73) Proprietor: **Kodimule, Shyam Prasad**
**562106 Bangalore (IN)**

(72) Inventor: **Kodimule, Shyam Prasad**
**562106 Bangalore (IN)**

(74) Representative: **Banse, Klaus-Dieter**
**Banse & Steglich Patentanwälte PartmbB**
**Herzog-Heinrich-Straße 23**
**80336 München (DE)**

(56) References cited:
**WO-A2-2014/138426**　　**CA-A1- 2 358 013**
**CN-A- 102 920 696**　　**JP-A- 2011 184 351**

• EVANS SHIRLEY F ET AL: "Mango supplementation improves blood glucose in obese individuals.", NUTRITION AND METABOLIC INSIGHTS 2014, vol. 7, 3 July 2014 (2014-07-03), pages 77-84, XP002754241, ISSN: 1178-6388
• EDRALIN A. LUCAS ET AL: "Mango modulates body fat and plasma glucose and lipids in mice fed a high-fat diet", BRITISH JOURNAL OF NUTRITION, vol. 106, no. 10, 28 June 2011 (2011-06-28), pages 1495-1505, XP055249422, UK ISSN: 0007-1145, DOI: 10.1017/S0007114511002066
• LING L T ET AL: "Standardised Mangifera indica extract is an ideal antioxidant", FOOD CHEMISTRY, ELSEVIER LTD, NL, vol. 113, no. 4, 15 April 2009 (2009-04-15), pages 1154-1159, XP025674926, ISSN: 0308-8146, DOI: 10.1016/J.FOODCHEM.2008.09.004 [retrieved on 2008-09-08]
• C.M. AJILA ET AL: "Characterization of bioactive compounds from raw and ripe Mangifera indica L. peel extracts", FOOD AND CHEMICAL TOXICOLOGY., vol. 48, no. 12, 1 December 2010 (2010-12-01), pages 3406-3411, XP055249553, GB ISSN: 0278-6915, DOI: 10.1016/j.fct.2010.09.012

Description

## FIELD OF THE INVENTION

The invention generally relates to herbal compositions. More particularly, the invention relates to herbal compositions derived from *Mangifera indica* and methods for their use in the treatment and management of obesity and/or weight.

## BACKGROUND

Obesity is a condition manifesting almost directly as a consequence of a modern day lifestyle that encompasses sedentary work-culture, high fat, calorie-rich diet, dearth of regular exercise or physical activity, addiction to habit forming substances such as tobacco and alcohol and high day-to-day stress levels. Obesity has reached epidemic proportions globally, with more than 1 billion adults overweight - at least 300 million of them clinically obese - and is a major contributor to the global burden of chronic disease and disability. Ischemic heart disease and cardiovascular diseases are the conditions, often referred to as lifestyle diseases, have obesity as one of their root causes. Ischemic heart disease is the number one cause of death in the world today, according the World Health Organization (WHO). Cardiovascular diseases (CVDs) have killed nearly 17 million people in the year 2011 which amounts to 3 in every 10 deaths. CVDs are among the top causes of death, in India as well, as per the WHO. The importance of managing obesity is, thus, evident.

Often coexisting in developing countries with under-nutrition, obesity is a complex condition, with serious social and psychological dimensions, affecting virtually all ages and socioeconomic groups. Obesity and being overweight pose a major risk for other serious chronic diseases, including type 2 diabetes, hypertension, stroke and certain forms of cancer. The health consequences range from increased risk of premature death, to serious chronic conditions that reduce the overall quality of life.

It is, therefore, safe to state that managing obesity would substantially aid in reducing global mortality, increasing life expectancy and increasing the quality of life. Dietary changes, exercise and activity, behavioral change, prescription weight-loss medications and weight-loss surgery are the common treatment arms for managing obesity. The treatment method to be undertaken often depends on the preferred choice of an individual undergoing treatment as well as the level of obesity.

The preferred treatment modality for weight loss is dieting and physical exercise. However, due to busy schedules and sedentary lifestyles, following-up with the first two methods seems to be practiced in an irregular manner. Weight loss surgery, on the other hand, is ruled out by a host of the population due to high costs involved. Therefore, there is a gradual shift towards an increase in the use of drugs.

Drugs used for weight-loss generally alter one of the fundamental processes of the human body such as weight regulation by altering appetite, metabolism or absorption of calories. Orlistat is the only anti-obesity medication that is approved for long-term use by the FDA. It reduces the intestinal fat absorption by inhibiting the pancreatic enzyme lipase. Rimonabant and Sibutramine are the other drugs that had initially been approved for the treatment of obesity, but were banned eventually due to safety concerns. Because of the potential side effects, it is recommended that anti-obesity drugs only be prescribed for obesity where it is hoped that the benefits of the treatment outweigh its risk.

In view of the above, nutrition-based interventions are being hailed as an inexpensive alternative to aid weight loss and to manage weight in a better way. Medicinal herbal supplements, being cost-effective and exerting less to no toxic side-effects, are being extensively utilized due to their effectiveness in managing many chronic disorders.

The inventors of the present disclosure, therefore, envisage a cost-effective and safe herbal composition or a dietary supplement which is used for the management of obesity based on the extract of *Mangifera indica*. *Mangifera indica* is a large evergreen tree belonging to Anacardiaceae *which is native* to tropical Asia and has been cultivated in the Indian subcontinent for over 4,000 years. Chemical constituents of *Mangifera indica* are always of an interest especially the polyphenolics, flavonoids and triterpenoids. The bark is reported to contain protocatechic acid, catechin, mangiferin, alanine, glycine, γ-aminobutyric acid, kinic acid, shikimic acid etc. *Mangifera indica* leaves extract revealed the presence of steroids, flavonoid, reducing sugar and cardiac glycosides in the hexane extract; anthraquinone, tannin and reducing sugar in the ethyl acetate extracts and saponin, steroids, tannin, flavonoid, reducing sugars and cardiac glycosides in the methanolic extracts [Aiyelaagbe et al.,2009]. Fruit skin has been found to be a good source of phytochemicals, such as polyphenols, carotenoids, vitamin E and vitamin C [Ajila et al., 2007a].

The relative biochemical complexity of *Mangifera indica* stem bark and leaf extract may produce a pleiotropic action on several lipid and carbohydrate metabolism targets simultaneously, making them as multifunctional botanical therapeutics useful in weight control. They may also provide effective biochemical tools for studying the complex relationships between energy balance, adiposity and endocrine function [Moreno et al., 2005]. Also, mango fruit skin and flesh extracts affect adipogenesis in 3T3-L1 cells [Taing et al., 2012].

Mangiferin a xanthone glycoside major bio-active constituent, isomangiferin, tannins & gallic acid derivatives [Shah et al., 2010]. It is a major C-glucosylxanthone from *M indica* stem bark, leaves, heartwood, roots and fruits [Wauthoz et al., 2007]. Mangiferin, a natural polyphenol, has been shown to have hypolipidemic effect in rat and mouse [Guo et al., 2011]. Presence of mangiferin in the herbal composition could be one of the factors for the anti-obesity action. Hence in view of this herbal composition containing extract of *Mangifera indica* was evaluated for its anti obesity property.

## SUMMARY OF THE INVENTION

It is an object of the invention to provide a composition for use in managing obesity in a subject comprising administering to the subject a composition comprising an extract of Mangifera extract in an amount effective to manage obesity and/or weight in the subject.

Subject of the invention are compositions and uses according to claims 1 to 11.

Subject of the invention is a composition comprising an extract of Mangifera extract for use in treating obesity, wherein the extract is an extract of Mangifera indica bark, leaf and fruit skin. As used herein, the term "treating" also relates to prevention of obesity.

Subject of the invention is a non therapeutic use of a composition comprising an extract of Mangifera for managing weight in a subject, wherein the extract is an extract of Mangifera indica bark, leaf and fruit skin, comprising administering to the subject in an amount effective to manage weight in the subject. The term "managing weight" especially means achieving weight loss or preventing weight gain.

In some aspects of the invention, the amount effective to manage obesity and/or weight in the subject ranges between 50-500 mg/kg body weight.

In some aspects of the invention, the amount effective to manage obesity and/or weight in the subject ranges between 50-500 mg/kg body weight.

In some aspects of the invention, managing obesity and/or weight in the subject comprises decreasing at least one of appetite, body weight gain, metabolism and absorption of calories.

In some aspects of the invention, administering the composition modulates at least one of insulin levels, lipase levels and lipid peroxidation.

In some aspects of the invention, administering the composition modulates at least one of serum glucose, total cholesterol, triglyceride, HDL-c and LDL-c levels.

In some aspects of the invention, the composition comprises an extract of Mangifera indica bark, leaf and fruit skin in a ratio of 80:10:10 respectively.

In some aspects of the invention, the composition comprises, by weight, 29% flavonoids and 19% polyphenols.

In some aspects of the invention, the composition comprises, by weight, 22% mangiferin, 4% catechin, 3% epicatechin, and 0.4% quercitin dihydrate.

In some aspects of the invention, the composition inhibits super oxide dismutase in the subject.

In some aspects of the invention, the composition is administered as a powder, pill, tablet, pellet, capsule, thin film, solution, spray, syrup, linctus, lozenge, pastille, chewing gum, paste, vapor, suspension, emulsion, ointment, cream, lotion, liniment, gel, drop, topical patche, buccal patch, bead, gummy or injection.

It is a further object of the invention to provide a composition for use in managing obesity and/or for managing weight in a subject, wherein the composition comprises by weight, 22% mangiferin, 4% catechin, 3% epicatechin, and 0.4% quercitin dihydrate.

In some aspects of the invention, the composition is formulated as a powder, pill, tablet, pellet, capsule, thin film, solution, spray, syrup, linctus, lozenge, pastille, chewing gum, paste, vapor, suspension, emulsion, ointment, cream, lotion, liniment, gel, drop, topical patch, buccal patch, bead, gummy or injection.

## BRIEF DESCRIPTION OF THE DRAWINGS

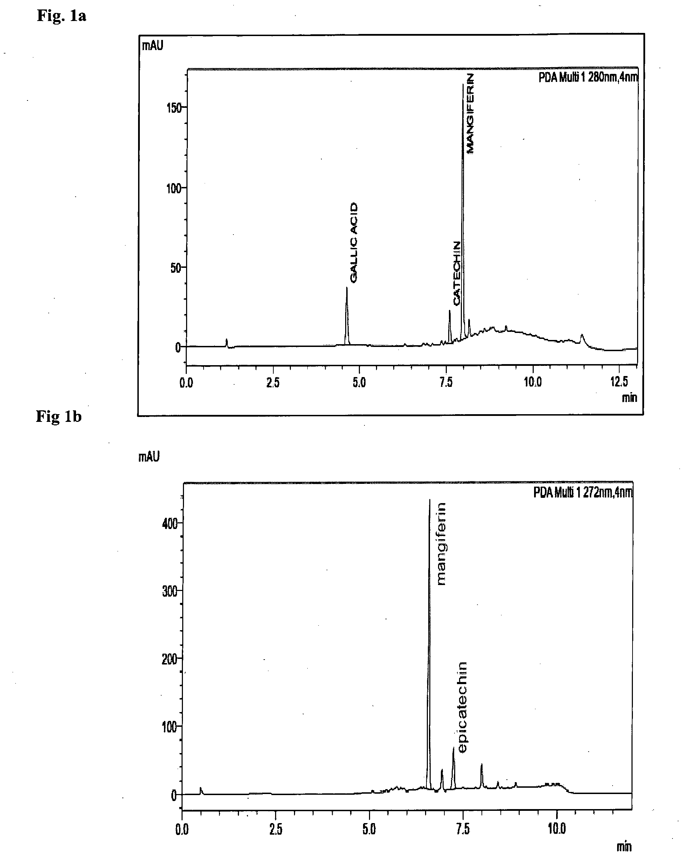

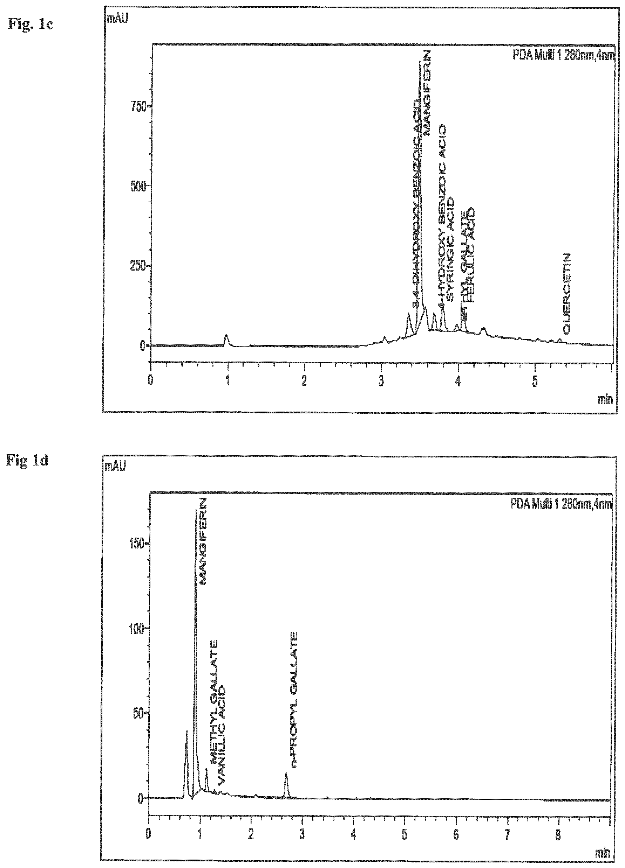

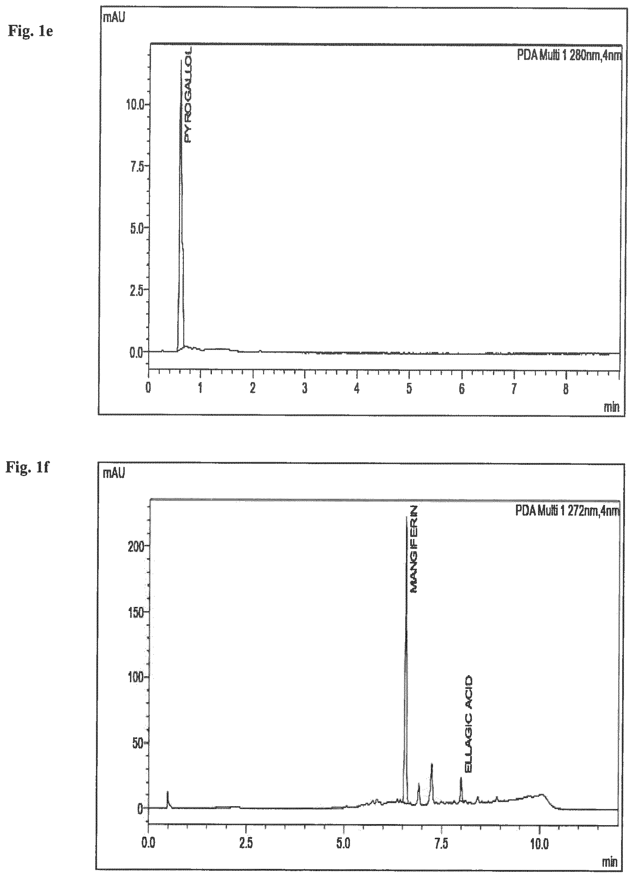

Fig. 1 shows HPLC chromatograms of herbal composition showing phenolic and flavonoidal constituents.

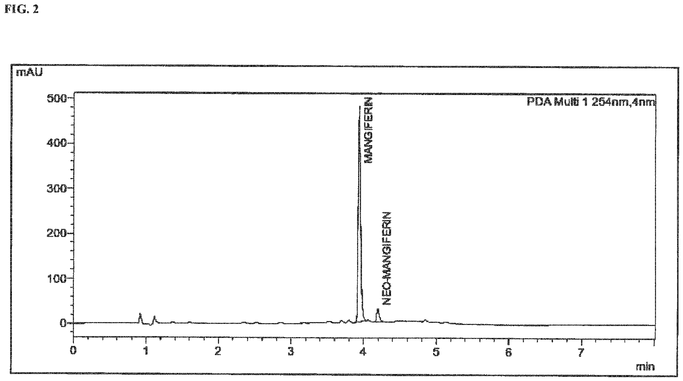

Fig. 2 shows a HPLC chromatogram of a herbal composition showing mangiferin peak.

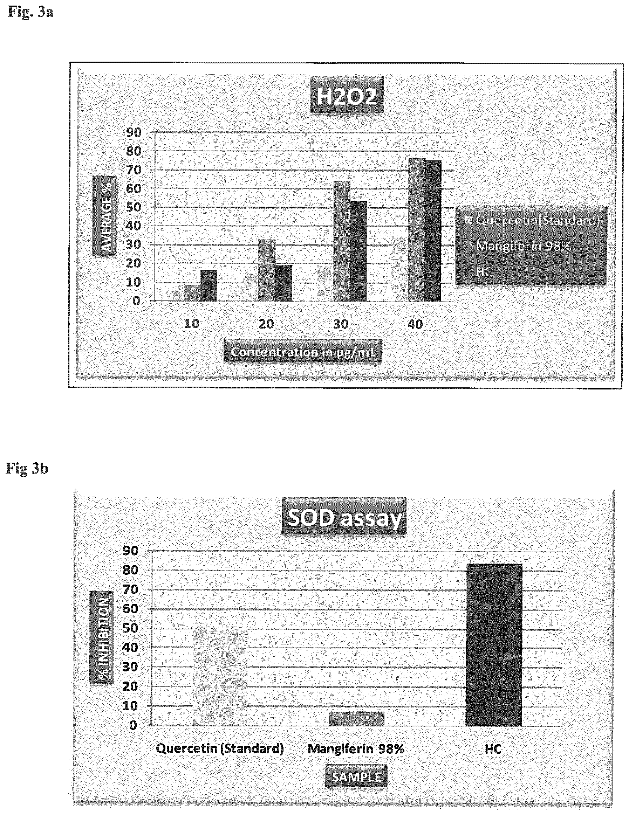

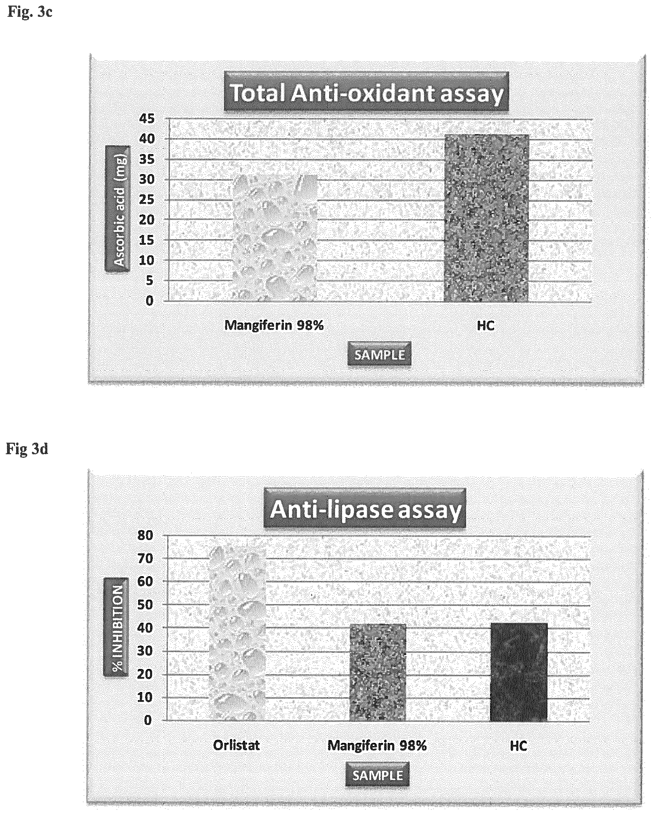

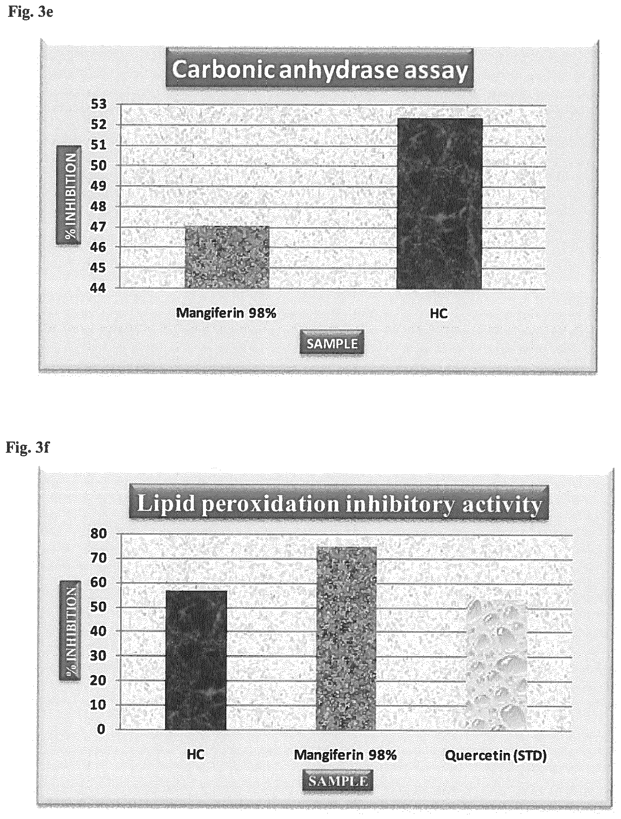

Fig 3 shows the in-vitro evaluation of $H_2O_2$ scavenging, SOD, anti-oxidant, Anti-lipase, lipid peroxidation inhibitory and Carbonic anhydrase activity."

## DEFINITIONS

The term "about" as used herein refers to a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length. In particular embodiments, the terms "about" or "approximately" when preceding a numerical value indicates the value plus or minus a range of 15%, 10%, 5%, or 1%, or any intervening range thereof.

As used herein, the phrase *"Mangifera indica* material" refers to material from the *Mangifera indica* plant, which is bark, leaves and fruit skin.

As used herein, the phrase "managing obesity" comprises treating or preventing obesity.

## DETAILED SPECIFICATION

The invention generally relates to herbal compositions and methods for their use in a variety of therapeutic and preventative applications. More particularly, the invention relates to compositions comprising *Mangifera indica* extract and methods for their use in the management of obesity and/or for managing weight.

The herbal compositions of the present invention may be administered by various routes including but not limiting to topical, oral, buccal, sub-lingual, parenteral, rectal, and inhalation. The compositions may be in a dosage form that includes but is not limited to powders, pills, tablets, pellets, capsules, thin films, solutions, sprays, syrups, linctuses, lozenges, pastilles, chewing gums, pastes, vaporizers, suspensions, emulsions, ointments, creams, lotions, liniments, gels, drops, topical patches, buccal patches, beads, gummies, gels, sols, injections and the like. Typically, the composition comprises at least one pharmaceutically acceptable excipient.

The herbal compositions of the present invention find use in a variety of therapeutic and preventive applications. In some embodiments of the invention, the herbal compositions are for use in administration, or non-therapeutically administered, to a subject for preventing oxidation (antioxidant activity). In other embodiments of the invention, the herbal compositions find use in treating, preventing or managing obesity and/or weight. The phrase "managing obesity and/or weight" as used herein refers to preventing or reducing body fat, preventing or reducing weight gain, preventing or reducing the absorption of fat in the diet, reducing body mass index (BMI), reducing abdominal fat, preventing weight gain from a high fat diet and combinations thereof. In embodiments of the invention, herbal compositions are for use in administration, or non-therapeutically administered, in the management of hyperlipidemic conditions, including but not limited to, reducing serum low density lipids, cholesterol and triglycerides, raising high density lipids (HDL) and combinations thereof. In other embodiments of the invention, the herbal compositions are for use in administration, or non-therapeutically administered, to prevent or reduce the accumulation of fat in the liver. In other embodiments, the herbal compositions of the invention are for use in administration, or non-therapeutically administered, to reduce oxidative stress or regulating blood glucose levels. The term "reduce" as used herein refers to any measurable decrease that is produced as a result of administering the compositions of the invention, relative to the absence of such administration. Significantly, the compositions of the invention are non-toxic and non-mutagenic. Similarly, the term "increase," or "raise," refers to any measurable increase that is produced as a result of administering the compositions of the invention, relative to the absence of such administration.

An aspect of the invention concerns portions of *Mangifera indica* that are used in preparation of the herbal composition. Accordingly, the herbal composition are prepared from Mangifera indica bark, leaf and fruit skin. In yet another embodiment of the invention, the compositions are prepared from *Mangifera indica* bark, leaves and fruit skin in a ratio of about 80:10:10 respectively.

Another aspect of the invention concerns the dosage of the herbal composition that is used. The dosage of the composition will depend on the nature of the condition to be treated or managed, and the degree or advancement of the condition being addressed. Some non-limiting examples of dosages for use with the invention inlude 50-500 mg/kg body weight. In one aspect of the invention, the dosage is 500 mg/kg body weight. The dosages disclosed herein may be administered between one and four times daily. In one aspect of the invention, the selected dosage is administered three times a day.

Another aspect of the invention concerns methods for making the subject herbal compositions. In some aspects, the herbal compositions are made by ethanol extraction. In other aspects of the invention, the compositions are made by aqueous extraction. In still other embodiments, the compositions of the invention are prepared by compressing a *Mangifera indica* material.

## EXAMPLE 1 - EXTRACTION PROCEDURE OF HERBAL COMPOSITION

Dried parts of *Mangifera indica* bark, leaf and fruit skin (80:10:10 ratio) were cut in to small pieces, approximately 10 mm in length, to form a 100 kg mass. This mass was pulverized to form a coarse powder in a hammer mill and stacked in a vertical 1.0 KL extractor. The lower end of the extractor contained a perforated plate on which a filtration cloth was fixed. The bottom of the extractor was connected to a transfer pump input and the output of the transfer pump was connected to a T bend. One end was connected to the extractor top for circulation of the extraction mass during the extraction period and the other end of the T bend was connected to a receiver tank.

The above-mentioned coarse powder was initially extracted with six bed volumes of 70% v/v ethyl alcohol. The extraction was carried out at 75-78°C for about 7-8 hours with continuous circulation of the extract with a transfer pump. After completion of extraction, the extract was filtered through a 5 micron SS candle filter and the resultant clear extract was collected in a receiver tank. The bed was re-extracted by adding 4 bed volumes of 70% ethyl alcohol 3 more times at a temperature of 75-78°C for about 7-8 hours. All the extracts were then collected in a receiver tank and the combined extract was concentrated in a reactor under vacuum at 80-85°C till the extract mass- total dissolved solids (TDS) reached 60-70%. The extract mass was further dried in a vacuum tray drier at 80-85°C until the completion of drying. The yield of the extract, the herbal composition (HC) was found to be 14±0.5 w/w%. The extract was further subjected to phyto-chemical analysis through spectrophotometric and HPLC estimations. The studies confirmed that the HC is associated with good percentage of antioxidant constituents.

Table 1 Results of phyto-chemical analysis

| S. N. | Phyto-constituents | Percentage | Analysis Method |
|---|---|---|---|
| 1 | Total flavonoids | 28.56±0.93w/w% | Spectrophotometric |
| 2 | Total polyphenols | 18.9±1.8 w/w% | Spectrophotometric |

The composition of the total flavonoids was determined by the HPLC method and the following results were obtained:

Table 2 Composition of the determined flavonoids

| S. N. | Constituents | Percentage |
|---|---|---|
| 1 | Mangiferin | 21.5±0.82 w/w% |
| 2 | (+)-Catechin | 3.84±0.31 w/w% |
| 3 | (-)-Epicatechin | 2.67±0.25 w/w% |
| 4 | Quercetin dihydrate | 0.43±0.06 w/w% |

The composition of the total polyphenols was determined by the HPLC method and the following results were obtained:

Table 3 Composition of the determined polyphenols

| S.N. | Constituents | Percentage |
|---|---|---|
| 1 | Gallic acid | 4.5±0.42 w/w% |
| 2 | 3, 4-Dihydroxy benzoic acid | 0.93±0.23 w/w% |
| 3 | Methyl gallate | 1.2±0.34 w/w% |
| 4 | Proplyl gallate | 2.31±0.73w/w% |
| 5 | Pyrogallol | 0.48±0.08 w/w% |
| 6 | Parahydroxy benzoic acid | 1.43±0.07 w/w% |
| 7 | Vanillic acid | 0.33±0.06 w/w% |
| 8 | Syringic acid | 0.82+0.13 w/w% |
| 9 | Ferulic acid | 0.12±0.02 w/w% |
| 10 | Ethyl gallate | 0.76±0.14 w/w% |
| 11 | Ellagic acid | 0.16±0.05 w/w% |

Table 4 Peak Table showing phenolic and flavonoids in HC

| Name | Ret. Time | Area | Height | Area % | Lambda max |
|---|---|---|---|---|---|
| Mangiferin | 3.936 | 1120914 | 479844 | 94.067 | 257/241/318/366/580 |
| (+)- Catechin | 7.598 | 58049 | 20738 | 9.623 | 203/278/366/657/622 |
| (-)-Epicatechin | 7.245 | 197931 | 62255 | 15.075 | 208/274/662/623 |
| Quercetin dihydrate | 5.314 | 29209 | 12881 | 1.038 | 254/370/199 |
| Gallic acid | 4.629 | 132932 | 36837 | 22.038 | 215/270/387/656/404 |
| 3, 4-Dihydroxybenzoic acid | 3.352 | 245290 | 75658 | 8.716 | 260/206/222/295/365 |
| Methyl gallate | 1.114 | 32395 | 13176 | 6.362 | 215/271 |
| Proplyl gallate | 2.680 | 53767 | 15055 | 10.559 | 216/273 |
| Pyrogallol | 0.598 | 37015 | 11721 | 100.000 | 202/257/240/315/362 |
| Parahydroxy benzoic acid | 3.683 | 127757 | 54283 | 4.540 | 256/315/198 |
| Vanillic acid | 1.269 | 4780 | 2177 | 0.939 | 204/260 |
| Syringic acid | 3.795 | 211448 | 79433 | 7.513 | 271/226 |
| Ferulic acid | 4.062 | 190504 | 74471 | 6.769 | 261/360 |
| Ethyl gallate | 3.975 | 46246 | 17396 | 1.643 | 261/245 |
| Ellagic acid | 7.990 | 43005 | 18447 | 6.750 | 253/366/662/623 |

## EXAMPLE 2 - UPLC ANALYSIS FOR HERBAL COMPOSITION

Reagents required

Mangiferin (98% purity) was procured from Sigma Aldrich. Chemicals required for the analysis Acetonitrile (ACN), Methanol (MeOH), Formic acid, double distilled water was of LC grade.

Solution Preparation

Around 10 mg of HC was weighed into a 10 mL standard flask. Approximately 3 mL of 70 % methanol (LC grade) was added. The mixture was sonicated for 10 minutes, and made up to the mark with the same solvent. The sample solution was filtered through a 0.20 $\mu$m a nylon filter.

Standard preparation

Around 10 mg of standard (98%) was weighed into a 10 mL standard flask and dissolved in 70 % methanol (LC grade). The mixture was sonicated for 10 minutes, and made up to mark with the same solvent. The sample solution was filtered through a 0.20 $\mu$m nylon filter.

UPLC Analysis of Herbal Composition

A sensitive and selective ultra performance liquid chromatography (UPLC) method was used for the fingerprint analysis of herbal composition. The LC system consisted of a Shimadzu LC MS/MS # 8040 system. The column used was SHIM-PACK XR-ODSIII, 5$\mu$m, 150X 2.1 mm with mobile phase consisting 0. 1% Formic acid in LCMS grade water: Acetonitrile with isocratic gradient system at run time of 8 min. The flow rate was maintained at 0.35 mL/min at 25 $\pm$ 2°C. The eluate was monitored at 254 nm. Retention time was as mentioned in Table 5.

Table 5 Optimized Chromatographic Conditions

| S.NO | PARAMETERS | SPECIFICATION |
|---|---|---|
| 1 | Instrument | LC MS/MS # 8040 |
| 2 | Detector | SPD-M20A PDA |

(continued)

| S.NO | PARAMETERS | SPECIFICATION |
|---|---|---|
| 3 | Mobile phase A:B | 0.1% Formic acid in LCMS grade water : Acetonitrile |
| 4 | Column | SHIM-PACK XR-ODSIII, 5μm, 150X 2.1 mm |
| 5 | Pump | Nexera X2, LC-30AD Shimadzu |
| 6 | Wavelength | 254 nm |
| 7 | Flow rate | 0.35 mL / min |
| 8 | Volume of injection | 1 μL |
| 9 | Run time | 8 min |

Table 6 Peak Table showing mangiferin in Herbal composition

| Name | Ret. Time | Area | Height | Area % | Lambda max |
|---|---|---|---|---|---|
| Mangiferin | 3.936 | 1120914 | 479844 | 94.067 | 257/241/318/366/580 |
| Neo-mangiferin | 4.195 | 70695 | 29923 | 5.933 | 259/243/319/364/579 |
| Total | | 1191609 | 509767 | 100.00 | |

## EXAMPLE 3 - IN-VITRO EVALUATION OF HERBAL COMPOSITION

Antioxidant activity studies:

Hydrogen peroxide ($H_2O_2$) scavenging activity:

Procedure:

Hydrogen peroxide scavenging activity of the HC was estimated in this study where initially, HC solutions were prepared at different concentrations in distilled water and the resultant solutions were mixed in 0.6 ml of 40 mM $H_2O_2$ solution prepared in phosphate buffer (0.1 M pH 7.4) and incubated for 10 min. The absorbance was measured at 230 nm against a blank solution containing $H_2O_2$. Similar procedure was repeated for HC, Mangiferin and Quercetin. The amount of hydrogen peroxide radical inhibited was calculated using the following equation. The result is expressed as % $H_2O_2$ radical scavenging activity.

$$H_2O_2 \text{ radical scavenging activity} = \left\{ \frac{(\text{Abs control} - \text{Abs sample}) \times 100}{(\text{Abs control})} \right\}$$

where:

Abs control - Absorbance of $H_2O_2$ radical
Abs sample - Absorbance of sample extract/ standard (the HC, mangiferin and quercetin)

Observation:

Table 7 H$_2$O$_2$ scavenging activity

| H$_2$O$_2$scavenging activity | | | | |
|---|---|---|---|---|
| Samples | Concentration in μg/ml | | | |
| | 10 | 20 | 30 | 40 |
| Quercetin (Standard) | 5.6 | 14.55 | 18.86 | 33.96 |
| Mangiferin 98% | 8.49 | 33.01 | 64.15 | 76.41 |
| HC | 16.43 | 19.17 | 53.42 | 75.34 |

Conclusion: The HC exhibited good H$_2$O$_2$ scavenging activity in concentration dependent manner.

Superoxide Dismutase (SOD) Activity:

Procedure:

The superoxide anion scavenging activity of the HC was measured in this test. 1 ml (156 μM) of Nitro blue tetrazolium (NBT), 1 ml (468 μM) of nicotinamide adenine dinucleotide (NADH) and 3 ml of the HC at 100 μg/ml concentration were admixed in phosphate buffer (pH 7.4). The reaction was started by adding 100 μl of phenazine methosulfate (PMS) (0.18mg/ml) and the mixture was incubated at 25°C for 5 min followed by measuring the absorbance at 560 nm. Similar solutions were prepared for quercetin and mangiferin and the percentage of inhibition was calculated by using the formula provided herein-below; the result was expressed as % superoxide dismutase scavenging activity.

$$\% \text{ superoxide dismutase inhibition} = \left\{ \frac{(\text{Abs control} - \text{Abs sample}) \times 100}{\text{Abs control}} \right\}$$

where:

Abs control - Absorbance of control
Abs sample - Absorbance of sample extract/ standard (the HC, mangiferin and quercetin)

Observation:

Table 8 SOD activity results

| SOD activity | |
|---|---|
| Sample Concentration in μg/ml | % Inhibition |
| Quercetin (Standard) | 51.03 |
| Mangiferin 98% | 7.50 |
| HC | 83.40 |

Conclusion: HC exhibited significant SOD activity in comparison with 98% Mangiferin and quercetin.

Total antioxidant assay:

Procedure:

The total antioxidant activity of the HC was determined by the phosphomolybdenum method where 0.3 ml of the HC (100μg/ml) was combined with 3ml of a reagent solution (0.6M sulfuric acid, 28mM sodium phosphate and 4mM ammonium molybdate). The resultant reaction mixture was incubated at 95°C for 90 min and cooled to room temperature. The absorbance of the solution was measured at 695 nm against a blank solution (water). The total antioxidant capacity was expressed as the milligram equivalents of ascorbic acid.

Observation:

Table 9 Total antioxidant assay

| Total antioxidant assay | |
| --- | --- |
| Sample Concentration $\mu g$/ml | mg equivalent ascorbic acid |
| Mangiferin 98% | 31.23 |
| HC | 41.06 |

Conclusion: The total antioxidant capability of the HC was found to be 41.06 mg equivalent ascorbic acid. The HC exhibited good antioxidant property. The antioxidant ability of HC was confirmed through hydrogen peroxide radical scavenging activity, superoxide radical scavenging activity and the total antioxidant capacity.

*In vitro* Anti-obesity studies:

Anti-lipase assay:

Procedure:

A suspension containing 1% (V/V) of triolein and 1% (V/V) of tween 40 in 0.1M phosphate buffer (pH-8) was prepared and emulsified. The assay was initiated by adding $800\mu l$ of the Triolein emulsion to $200\mu l$ of Porcine Pancreatic Lipase (0.5 g Pancreatic Lipase in 15 ml of 0.1 M phosphate buffer at pH-8) and 200 $\mu l$ of the HC extract (100 $\mu g$/ml concentration). The resultant mixture was mixed and the absorbance was measured immediately at 450 nm ($T^0$). The mixtures were then incubated at 37 °C for 30 min and the absorption was measured at 450 nm ($T^{30}$).

$$\text{Inhibition \%} = \left\{ \frac{A(\text{cont}) - A(\text{test})}{A(\text{cont})} \times 100 \right\}$$

where:

A(cont) - is the absorbance of the control ($T_C^0$-$T_C^{30}$)
A(test) - is the absorbance of the sample test /standard ($T^0$-$T^{30}$) (the HC, mangiferin and quercetin)

Observation:

Table 10 Anti-lipase assay results

| Anti-lipase assay | |
| --- | --- |
| Sample Concentration: 1.2 mg/ml | % Inhibition |
| Orlistat | 75.27 |
| Mangiferin 98% | 41.78 |
| HC | 42.30 |

Conclusion: The HC showed good anti-lipase property.

Carbonic Anhydrase Assay:

Procedure:

The HC at 250 μg/ml and 98% Mangiferin were independently mixed in 1.4 ml of tris buffer and 2.5 ml of carbonic anhydrase. The resultant mixtures were incubated at room temperature for 5 min. After incubation, 1 ml of 4-NPA was added to the mixtures and further incubated for 15-20 min at 37 °C. The absorbance of the reaction mixtures were measured at 400 nm.

$$\text{Inhibition \%} = \left\{ \frac{A(cont) - A(test)}{A(cont)} \times 100 \right\}$$

where:

A (cont) - is the absorbance of the control
A (test) - is the absorbance of the test sample /standard

Observation:

Table 11 Carbonic Anhydrase test results

| Carbonic Anhydrase | |
| --- | --- |
| Sample Concentration: 100μg | % inhibition |
| Mangiferin 98% | 47.06 |
| HC | 52.31 |

Conclusion: The HC showed enhanced anti-lipase property as compared to Mangiferin.

Lipid peroxidation inhibitory assay:

Procedure:

A modified Thiobarbituric acid reactive species (TBARS) assay (Ohkowa et al., 1979) is used to measure lipid peroxidation formed using egg yolk homogenate as lipid rich media (Ruberto et al., 2000).
Take 0.5ml of egg homogenate (10%in distilled water) and 0.1 ml of test sample in the concentration range 100-500μ g/ml in a test tube and volume made up to 1 ml by adding distilled water. Finally add 0.05 ml ferrous sulphate (0.07M) to the above mixture and incubate for 30 min to induce the lipid peroxidation. Thereafter add 1.5 ml of 20% acetic acid (pH 3.5) and 1.5 ml of (0.8%in 1.1% SDS) TBA and 0.05 ml (20%) TCA then heated in boiling water bath for 60 min. After cooling 5 ml of butanol will be added to all the test tubes and centrifuged at 3000 rpm for 10 min. Measure the absorbance of the organic upper layer at 532 nm. The percentage inhibition of lipid peroxidation calculated from the formula,

$$\text{\% Inhibition} = \frac{(Ab_{control} - Ab_{test\ sample})\ *100}{Ab_{control}}$$

where:

Ab (cont) - is the absorbance of the control
Ab (test sample) - is the absorbance of the test sample /standard

Table 12 Lipid peroxidation inhibitory assay test results

| Lipid peroxidation inhibitory assay | |
|---|---|
| Sample Concentration: 100-500μ g/ml | % inhibition |
| HC | 56.52 |
| Mangiferin 98% | 74.9 |
| Quercetin (STD) | 53.44 |

Conclusion: The HC showed enhanced lipid peroxidation property as compared to Quercetin. Herbal composition possesses good hypolipidemic and anti-obesity activity, which may be due to its antioxidant and free radical scavenging potential.

Example Acute oral toxicity study in rats with the HC

Single-dose oral toxicity of the HC was evaluated in albino Wistar rats. A limit test was performed in which female rats received a single oral administration of the HC at a dose of 2000 mg/kg body weight. Following dosing, the limit test rats were observed daily and weighed weekly. A gross necropsy examination was performed on all limit test animals at the time of scheduled euthanasia (day 14). No mortality occurred during the duration of the limit test. Further, no significant gross internal findings were observed at necropsy on study day 14.

Conclusion: Under the conditions of this test, the acute oral lethal dose ($LD_{50}$) of the HC was estimated to be greater than 2000 mg/kg in rats.

Repeated Dose 28 Day Oral Toxicity Study of the HC

Repeated dose 28 day oral toxicity study, with the HC in rats followed by 14 day recovery period, was performed. The objective of the study was to assess the safety of the HC when administered orally to female and male rats. Another objective was to determine the target organ toxicity, no observed effect level (NOEL) and reversibility of signs of toxicity after the recovery period.

Wistar female and male rats were treated with the HC 250,500 and 1000 mg/kg/b.w for 28 days by oral gavage, followed by 14 day recovery period.

Observations: 1) all the male and female animals from control and all the treated dose groups up to 1000 mg/kg survived throughout the dosing period of 28 days and the recovery period of 14 days; 2) no signs of intoxication were observed in male and female animals from different dose groups during the dosing period of 28 days and during the recovery period of 14 days; 3) male rats showed a significant decrease in body weight gain with 250 and 500 mg/kg b.w. of the HC and a significant decrease in body weight gain were seen with 500 and 1000 mg/kg b.w. of the HC in females when compared with the control on day 29. During the reversal period the animals restored to normal when compared with control reversal group on day 43; 4) food consumption of control and treated animals was found to be comparable throughout the dosing period of 28 days and the recovery period of 14 days; 5) haematological analysis conducted at the end of the dosing period on day 29 and at the end of recovery period on day 43, revealed no abnormalities attributable to the treatment; 6) biochemical analysis conducted at the end of the dosing period on day 29 and at the end of recovery period on day 43, revealed no attributable changes to the treatment, except there was a significant increase in high density lipoproteins (HDL) levels in female rats treated with 250, 500 and 1000 mg/kg/b.w. of the HC; 7) functional battery observation tests conducted at termination revealed no abnormalities; 8) urine analysis, conducted at the end of the dosing period in week 4 and at the end of recovery period in week 6,revealed no abnormality attributable to the treatment; 9) organ weight data of male and female sacrificed at the end of the dosing period and at the end of the recovery period was found to be comparable with that of respective controls, gross pathological examination did not reveal any abnormality; histopathological examination did not reveal any major abnormality except congestion of blood vessels in few organs.

Conclusion: Based on the above findings, the no observed adverse effect level (NOAEL) of the HC was found to be 1000 mg/kg/b.w. for both female and male Wistar rats wen given orally for 28 days followed by a 14 day recovery period.

**EXAMPLE 4 -Anti-obesity activity of the HC w.r.t Standard:**

Objective: The objective was to investigate the effect of the HC against high fat diet fed rats.

Procedure:

Animals: Male Wistar rats (160 to 200 g); No of animals: 36; Housing: 3/ cage, 12 hrs light/dark cycle; Temperature: $25 \pm 2°c$;

Model & Composition of the Diet: High fat diet in rats: 25% Lard, 5% Soyabean oil; 5% starch; 65% Normal commercial available rat feed;

Groups: Male Wistar rats were divided into six groups with six animals in each group

| Group | Diet |
|---|---|
| Group 01: | Normal feed + Vehicle (distilled water) |
| Group 02: | High fat diet |
| Group 03: | High fat diet + Standard drug (Orlistat 30 mg/kg) |
| Group 04: | High fat diet + HC (50 mg/kg) |
| Group 05: | High fat diet + HC (100 mg/kg) |
| Group 06: | High fat diet + HC (150 mg/kg) |

Observations:

Effect of the HC on body weight gain in male rats

Table 13 Results of the effect of the HC on body weight gain in male rats

| Groups | Final weight | Initial weight | Difference in body weight gain |
|---|---|---|---|
| Group I | $251.70 \pm 2.02$ | $181.70 \pm 1.99$ | $70.00 \pm 2.60$ |
| Group II | $292.50 \pm 1.81$ | $181.50 \pm 1.31$ | $111.0 \pm 2.20$***[a] |
| Group III | $261.80 \pm 2.53$ | $182.00 \pm 0.96$ | $79.82 \pm 2.58$***[b] |
| Group IV | $254.80 \pm 1.87$ | $182.30 \pm 1.99$ | $72.43 \pm 3.16$***[b] |
| Group V | $250.50 \pm 2.53$ | $183.30 \pm 0.88$ | $67.15 \pm 2.75$***[b] |
| Group VI | $259.20 \pm 6.06$ | $184.70 \pm 0.80$ | $74.50 \pm 5.77$***[b] |

Values are expressed in terms of SEM $\pm$ Mean. Data were analyzed by one way ANOVA followed by Dunnett's *t* test. Number of animals in each group n=6.
[a]Comparison made with control group.
[b]Comparison made with high fat diet group.***$P<0.001$.

Conclusion: The HC at all doses showed significant reduction ($P<0.001$) in body weight gain when compared to the high fat diet group.

Effect of the HC on the average food intake in male rats

Table 14 Results of the effect of the HC on the average food intake in male rats

| Groups | Average food intake g/rat |
|---|---|
| Group I | $17.21 \pm 0.38$ |
| Group II | $13.56 \pm 0.36$***[a] |
| Group III | $13.45 \pm 0.28$[nsb] |
| Group IV | $12.13 \pm 0.24$**[b] |

(continued)

| Groups | Average food intake g/rat |
|---|---|
| Group V | $11.26 \pm 0.24$***b |
| Group VI | $13.18 \pm 0.31$nsb |

Values are expressed in terms of SEM $\pm$ Mean. Data were analyzed by one way ANOVA followed by Dunnett's *t* test. Number of animals in each group n=6.
aComparison made with control group.
bComparison made with high fat diet group.
***P<0.001 ***P<0.001 **P<0.01 ns non significant.

Conclusion:

The HC at 50 and 100mg/kg b.w showed significant reduction (P<0.01, P<0.001) in food intake when compared to the high fat diet group.

Effect of the HC on liver organ weight and mesenteric, brown adipose tissue (BAT), perirenal fat pads and epididymal fat pad

Table 15 Results of the effect of the HC on liver organ weight and mesenteric, brown adipose tissue (BAT), perirenal fat pads and epididymal fat pad

| Groups | Liver | Mesenteric fat | BAT | Perirenal fat pad tissue | | Epididymal fat pad | |
|---|---|---|---|---|---|---|---|
| | | | | Right | Left | Right | Left |
| Group I | $2.8 \pm 0.01$ | $0.59 \pm 0.02$ | $0.13 \pm 0.01$ | $0.25 \pm 0.02$ | $0.21 \pm 0.01$ | $0.38 \pm 0.01$ | $0.33 \pm 0.02$ |
| Group II | $3.5 \pm 0.15$ ***a | $1.14 \pm 0.07$ ***a | $0.30 \pm 0.01$***a | $1.05 \pm 0.13$***a | $1.07 \pm 0.11$***a | $0.63 \pm 0.04$***a | $0.56 \pm 0.05$***a |
| Group III | $2.8 \pm 0.08$ ***b | $1.02 \pm 0.07$ nsb | $0.28 \pm 0.01$nsb | $0.77 \pm 0.08$*b | $0.77 \pm 0.08$*b | $0.55 \pm 0.02$nsb | $0.52 \pm 0.03$nsb |
| Group IV | $2.7 \pm 0.07$ ***b | $0.80 \pm 0.03$ **b | $0.17 \pm 0.01$***b | $0.49 \pm 0.01$***b | $0.44 \pm 0.03$***b | $0.51 \pm 0.01$*b | $0.42 \pm 0.01$*b |
| Group V | $2.8 \pm 0.03$ ***b | $0.83 \pm 0.03$ **b | $0.17 \pm 0.01$***b | $0.47 \pm 0.02$***b | $0.67 \pm 0.02$***b | $0.51 \pm 0.02$*b | $0.39 \pm 0.02$**b |
| Group VI | $2.9 \pm 0.02$ ***b | $0.84 \pm 0.03$ **b | $0.17 \pm 0.01$***b | $0.63 \pm 0.07$**b | $0.69 \pm 0.04$**b | $0.51 \pm 0.02$*b | $0.42 \pm 0.03$*b |

Data were analyzed by one way ANOVA followed by Dunnett's *t* test. Number of animals in each group n=6.
aComparison made with control group.
bComparison made with high fat diet group.
***P<0.001 **P<0.01 * P<0.05 ns non significant.

Conclusion: The HC at all doses showed significant reduction (P<0.001) in liver organ weight, Perirenal fat pads and BAT, Mesentric fat (P<0.001), epididymal fat pad (P<0.05) when compared to the high fat diet group.

Effect of the HC on serum glucose, total cholesterol, triglyceride, HDL-c and LDL-c levels (mg/dL)

Table 16 Results of the effect of the HC on serum glucose, total cholesterol, triglyceride, HDL-c and LDL-c levels (mg/dL)

| Groups | Glucose | Total cholesterol | Triglyceride | HDL-c | LDL-c |
|---|---|---|---|---|---|
| Group I | 81.63±2.96 | 47.53±1.15 | 52.61±2.14 | 22.61±0.56 | 14.40±1.51 |
| Group II | 114.20±7.63***[a] | 61.39±2.47***[a] | 104.6±4.16***[a] | 17.27±0.51***[a] | 23.20±2.96*[a] |
| Group III | 92.00±2.89**[b] | 50.91±0.62***[b] | 79.96±1.13***[b] | 21.72±1.22**[b] | 13.20±1.71**[b] |
| Group IV | 96.23±2.72*[b] | 48.96±1.90***[b] | 71.48±5.42***[b] | 23.77±0.99***[b] | 10.89±1.02***[b] |
| Group V | 82.37±2.82***[b] | 48.93±2.23***[b] | 55.92±2.03***[b] | 24.97±0.87***[b] | 12.77±1.75***[b] |
| Group VI | 96.70±0.96*[b] | 52.55±1.42**[b] | 79.77±5.3***[b] | 24.25±0.48***[b] | 12.35±2.13**[b] |

Data were analyzed by one way ANOVA followed by Dunnett's *t* test.
Number of animals in each group n=6.
[a]Comparison made with control group.
[b]Comparison made with high fat diet group.
***$P<0.001$ **$P<0.01$ * $P<0.05$

Conclusion:

The HC at 50 and 150 mg/kg b.w. showed a significant reduction ($P<0.05$), at 100mg/kg b.w. showed significant reduction ($P<0.01$) in glucose levels when compared to high fat diet group. The HC at 50 and 100 mg/kg b.w. showed a significant reduction ($P<0.001$), at 150mg/kg b.w. showed significant reduction ($P<0.01$) in total cholesterol and low density lipoproteins (LDL) levels when compared to high fat diet group. The HC at all doses showed a significant reduction ($P<0.001$) in total triglycerides and an increase in the HDL levels when compared to the high fat diet group (Group II).

Effect of the HC on atherogenic index

Table 17 Results of the effect of the HC on atherogenic index

| Group | Atherogenic Index |
|---|---|
| Group I | 1.11±0.08 |
| Group II | 2.57±0.18***[a] |
| Group III | 1.39±0.15***[b] |
| Group IV | 1.07±0.06***[b] |
| Group V | 0.96±0.06***[b] |
| Group VI | 1.17±0.08***[b] |

Data were analyzed by one way ANOVA followed by Dunnett's *t* test.
Number of animals in each group n=6.
[a]Comparison made with control group.
[b]Comparison made with high fat diet group.***$P<0.001$

Conclusion: The HC at all doses showed a significant reduction ($P<0.001$) in atherogenic index when compared to the high fat diet group.

Effect of the HC on liver triglyceride levels

Table 18 Results of the effect of the HC on liver triglyceride levels

| Group | Liver triglycerides |
|---|---|
| Group I | $63.85 \pm 0.42$ |
| Group II | $96.47 \pm 1.67$***[a] |
| Group III | $87.77 \pm 2.28$*[b] |
| Group IV | $78.57 \pm 1.93$***[b] |
| Group V | $86.47 \pm 2.11$**[b] |
| Group VI | $85.46 \pm 2.31$**[b] |
| Data were analyzed by one way ANOVA followed by Dunnett's *t* test.<br>Number of animals in each group n=6.<br>[a]Comparison made with control group.<br>[b]Comparison made with high fat diet group.<br>***P<0.001**P<0.01 * P<0.05 | |

Conclusion: The HC at 50 mg/kg b.w. showed a significant reduction (P<0.001), at 100 & 150mg/kg b.w. showed significant reduction (P<0.01) in liver triglycerides levels when compared to the high fat diet group.

Effect of the HC on insulin hormonal levels

Table 19 Results of the effect of the HC on insulin hormonal levels

| Group | Insulin (mU/L) |
|---|---|
| Group I | $0.27 \pm 0.023$ |
| Group II | $0.15 \pm 0.008$***[a] |
| Group III | $0.15 \pm 0.005$[nsb] |
| Group IV | $0.14 \pm 0.005$[nsb] |
| Group V | $0.22 \pm 0.016$*[b] |
| Group VI | $0.23 \pm 0.021$**[b] |
| Data were analyzed by one way ANOVA followed by Dunnett's *t* test.<br>Number of animals in each group n=6.<br>[a]Comparison made with control group.<br>[b]Comparison made with high fat diet group.<br>***P<0.001 **P<0.01* P<0.05 [ns] non significant. | |

Conclusion: The HC at 100 & 150mg/kg b.w. showed significant increase (P<0.05, P<0.01 respectively) in insulin levels when compared to the high fat diet group (Group II).

Effect of the HC on the fecal fatty acid estimation

Table 20 Effect of the HC on the fecal fatty acid estimation

| Group | Fecal fatty acid estimation |
|---|---|
| Group I | $1.0 \pm 0.05$ |
| Group II | $3.3 \pm 0.13$***[a] |
| Group III | $4.1 \pm 0.26$*[b] |

(continued)

| Group | Fecal fatty acid estimation |
|---|---|
| Group IV | $2.3\pm0.13^{**b}$ |
| Group V | $2.4\pm0.26^{**b}$ |
| Group VI | $3.1\pm0.26^{nsb}$ |

Data were analyzed by one way ANOVA followed by Dunnett's *t* test.
Number of animals in each group n=6.
[a]Comparison made with control group.
[b]Comparison made with high fat diet group.
***P<0.001 **P<0.01* P<0.05 [ns] non significant.

Conclusion: The HC at 50 & 100mg/kg b.w. showed significant reduction (P<0.01) in fecal fatty acids when compared to the high fat diet group.

Therefore, the *in vivo* anti-obesity studies of the HC revealed the following results.

The Herbal Composition:

- reduces body weight gain,
- reduces food intake
- removes abdominal fat tissue
- causes thermogenesis
- improves the HDL levels
- restores glucose levels
- Has liver protection.

## EXAMPLE 5 - CLINICAL STUDIES OF HERBAL COMPOSITION

A Prospective, Randomized, Double blind, Placebo controlled clinical trial to evaluate efficacy and safety of HC tablets in reducing the bodyweight in obese patients was carried out.

A total 30 numbers of subjects were included in the study as per inclusion criteria. Subjects were randomized and equal number of subjects were assigned in HC treatment group A and placebo treatment group B. The clinical study conducted for 56 days at Sampoorna Healthcare and Amruth Poly clinic, Bangalore, India. The study was performed in fulfillment with Declaration of Helsinki, 2002. The protocol and patient informed consent form was reviewed and approved by appropriate independent ethics committees of Sampoorna Healthcare and Amruth Poly Clinic before the starting of the study. Subjects were on a treatment for 56 days and examined at weeks 0, 2, 4, 8 of the study. Volunteers followed up for the two weeks after treatment period that was 10th week of the study. On 0th week, subjects were examined for physical examination, anthropometric measurements, quality of life assessment and multiple laboratory tests which include biochemical parameters, HbAlc measurements and hematology parameters as baseline data. During 2n[d] and 4th visits anthropometric measurements, physical examination and any adverse events were reported. On 8th week visit, physical examinations, anthropometric measurements, lipid profile, fasting blood glucose, HbA1c, quality of life assessment, adverse events, concomitant medications were recorded. All subjects were counseled for diet and exercise compliance at every visit. 10th week visit was meant to examine vital Signs, physical Examination, recording of adverse effects or serious adverse events, concomitant medications, weight, BMI & waist circumference, Waist to hip ratio and waist to height ratio. The subjects were asked to take one tablet containing 500 mg of either HC or placebo three times a day for 08 weeks before breakfast and meals.

Table 21 Primary and secondary outcome of HC treated group

| No. | Parameters | Visit 1 | Visit 4 | Difference in % |
|---|---|---|---|---|
| 1 | Body weight(kg) | $82.5\pm12.3$ | $77.25\pm12.9$ | 6.36 |
| 2 | Body mass index(BMI kg/m$^2$) | $30.96\pm1.76$ | $28.92\pm2.0$ | 6.59 |
| 3 | Waist circumference(cm) | $101.25\pm6.67$ | $94.92\pm5.9$ | 6.25 |

(continued)

| No. | Parameters | Visit 1 | Visit 4 | Difference in % |
|---|---|---|---|---|
| 4 | Hip circumference(cm) | 106.33±2.05 | 101.91±1.67 | 4.16 |
| 5 | Triglycerides(mg/dL) | 168.5±73.56 | 143.83±46.13 | 14.64 |
| 6 | Cholesterol(mg/dL) | 214.08±33.2 | 187.91±21.2 | 12.22 |
| 7 | LDL(mg/dL) | 138.27±34.9 | 104.33±25.73 | 24.55 |
| 8 | VLDL(mg/dL) | 33.7±14.71 | 28.76±9.22 | 14.66 |
| 9 | HDL | 42.1±8.4 | 54.81±5.2 | 30.19 |

Table 22 Primary and secondary outcome of Placebo treated group

| No. | Parameters | Visit 1 | Visit 4 | Difference in % |
|---|---|---|---|---|
| 1 | Body weight(kg) | 85.5±10.9 | 84.95±10.88 | 0.643 |
| 2 | Body mass index(BMI kg/m$^2$) | 32.33±1.88 | 32.04±1.81 | 0.90 |
| 3 | Waist circumference(cm) | 104.79±5.36 | 104.42±5.33 | 0.35 |
| 4 | Hip circumference(cm) | 108.83±1.70 | 108.46±1.73 | 0.34 |
| 5 | Triglycerides(mg/dL) | 188.5±75.4 | 185.75±74.68 | 1.46 |
| 6 | Cholestrol(mg/dL) | 204.91±43.8 | 201.16±43.2 | 1.83 |
| 7 | LDL(mg/dL) | 108.85±59.7 | 107.75±59.16 | 1.01 |
| 8 | VLDL(mg/dL) | 37.7±15.08 | 37.15±14.93 | 1.46 |
| 9 | HDL | 41.92±4 | 41.93±3.52 | 0.02 |

Clinical data suggest that HC is safe and has shown significant efficacy to reduce body weight and other obesity related complications.

**REFERENCES**

1. OECD Guideline for the testing of chemicals- Repeated Dose 90-day Oral Toxicity Study in Rodents; TG 408, 21st September 1998.

2. OECD Guideline No. 420, "Acute Oral Toxicity" -Fixed Dose Procedure 17th December 2001.

3. Chougule A., Manjunath A. (2012), Anti-obesity drugs of Bhava prakasha nighantu: The literary survey. IJRAP, 3 (5), 650-654.

4. Juliana Aparecida Severi , Zeila Pinheiro Lima , Hélio Kushima , Alba Regina Monteiro Souza Brito , Lourdes Campaner dos Santos, Wagner Vilegas , Clélia Akiko HirumaLima (2009) Polyphenols with Antiulcerogenic Action from Aqueous Decoction of Mango Leaves (Mangifera indica L.) . Molecules, 14, 1098-1110.

5. Taing M.W., Pierson J-T, Hoang V.L. T., Shaw P. N., Dietzgen R. G., Gidley M. J., Sarah J.,Thomson R, Monteith G. R. (2012) Mango fruit peel and flesh extracts affect adipogenesis in 3T3-L1 cells. Food Funct., 3, 828-836.

6. Moreno, D. A., Ripoll, C., Ilic, N., Poulev, A., Aubin, C., Raskin, I. (2005) Inhibition of lipid metabolic enzymes using Mangifera indica extracts. J. Food Agric. & Environ., Vol. 4(1): 21-26.

7. Ajila, C. M., Bhat, S. G., & Prasada Rao, U. J. S. (2007a). Valuable components of raw and ripe peels from two Indian mango varieties. Food Chemistry, 102, 1006-1011.

8. Aiyelaagbe O.O., Osamudiamen P. M. (2009) Phytochemical Screening for Active Compounds in Mangifera indica Leaves from Ibadan, Oyo State Plant Sciences Research, 2(1), 11-13.

9. Shah K.A., Patel M.B., Patel R.J., Parmar P.K. (2010) Mangifera Indica (Mango). Phcog. Rev., 4(7),42-48

10. Wauthoz N., Balde A. Balde E. S., Damme M. V., Duez P. (2007) Ethnopharmacology of Mangifera indica L. Bark and Pharmacological Studies of its Main C-Glucosylxanthone, Mangiferin. International Journal of Biomedical and Pharmaceutical Sciences, 1(2), 112-119.

11. Singh S. K., Sinha S. K., Prasad S.K., Kumar R., Bithu B. S., Kuma S. S., Singh P. (2011) Synthesis and evaluation of novel analogues of mangiferin as potent antipyretic. Asian Pacific Journal of Tropical Medicine, 866-869.

12. Gabino Garrido, Idania Rodeiro, Ivones Hernández, Gastón Garcia, Gema Pérez, Nelson Merino, Alberto Núñez-Sellés, René Delgado (2009) In vivo acute toxicological studies of an antioxidant extract from Mangifera indica L. (Vimang),32(1),53-8.

13. Fuchuan Guo, Conghui Huang, Xilu Liao, Yemei Wang, Ying He, Rennan Feng, Ying Li, Changhao Sun (2011) Beneficial effects of mangiferin on hyperlipidemia in high-fat-fed hamsters.Molecular Nutrition & Food Research. 55(12), 1809-18.

## Claims

1. A composition comprising an extract of Mangifera for use in treating obesity, wherein the extract is an extract of Mangifera indica bark, leaf and fruit skin.

2. A non-therapeutic use of a composition comprising an extract of Mangifera in managing weight in a subject, wherein the extract of Mangifera is an extract of Mangifera indica bark, leaf and fruit skin.

3. The composition for use of claim 1 or the use of claim 2, wherein the extract of Mangifera is present in an amount of 500mg.

4. The composition for use or the use of at least one of the preceding claims for altering at least one of appetite, body weight gain, metabolism or absorption of calories.

5. The composition for use or the use of at least one of the preceding claims for modulating at least one of insulin levels, lipase levels and lipid peroxidation.

6. The composition for use or the use of at least one of the preceding claims for modulating at least one of serum glucose, total cholesterol, triglyceride, HDL-c and LDL-c levels.

7. The composition for use or the use of at least one of the preceding claims, wherein the extract of Mangifera indica bark, leaf and fruit skin is an extract of Mangifera indica bark, leaf and fruit skin in a ratio of 80:10:10 respectively.

8. The composition for use or the use of at least one of the preceding claims, wherein the composition comprises, by weight, 29% flavonoids and 19% polyphenols.

9. The composition for use or the use of at least one of the preceding claims, wherein the composition comprises, by weight, 22% mangiferin, 4% catechin, 3% epicatechin, and 0.4% quercitin dihydrate.

10. The composition for use or the use of at least one of the preceding claims, wherein the composition inhibits super oxide dismutase in the subject.

11. The composition for use or the use of at least one of the preceding claims, wherein the composition is a powder, pill, tablet, pellet, capsule, thin film, solution, spray, syrup, linctus, lozenge, pastille, chewing gum, paste, vapor, suspension, emulsion, ointment, cream, lotion, liniment, gel, drop, topical patch, buccal patch, bead, gummy or injection.

## Patentansprüche

1. Zusammensetzung umfassend einen Mangos-Auszug zur Verwendung bei der Behandlung von Fettleibigkeit, wobei der Auszug ein Auszug aus Mango-Borke, -Blatt und -Fruchtschale ist.

2. Nicht-therapeutische Verwendung einer Zusammensetzung, die einen Mangos-Auszug umfasst, bei der Regelung des Gewichts eines Subjekts, wobei der Mangos-Auszug ein Auszug aus Mango-Borke, -Blatt und -Fruchtschale ist.

3. Zusammensetzung zur Verwendung nach Anspruch 1 oder Verwendung nach Anspruch 2, wobei der Mangos-Auszug in einer Menge von 500 mg vorliegt.

**4.** Zusammensetzung zur Verwendung oder Verwendung nach wenigstens einem der vorhergehenden Ansprüche zur Änderung wenigstens eines von Appetit, Körpergewichtszunahme, Metabolismus oder Absorption von Kalorien.

**5.** Zusammensetzung zur Verwendung oder Verwendung nach wenigstens einem der vorhergehenden Ansprüche zum Einstellen wenigstens eines von Insulinspiegeln, Lipasespiegeln und Lipidperoxidation.

**6.** Zusammensetzung zur Verwendung oder Verwendung nach wenigstens einem der vorhergehenden Ansprüche zum Einstellen wenigstens eines der Spiegel von Blutglukose, Gesamtcholesterin, Triglyceriden, HDL-C und LDL-C.

**7.** Zusammensetzung zur Verwendung oder Verwendung nach wenigstens einem der vorhergehenden Ansprüche, wobei der Auszug aus Mango-Borke, -Blatt und -Fruchtschale ein Auszug aus Mango-Borke, -Blatt und -Fruchtschale in einem Verhältnis von jeweils 80:10:10 ist.

**8.** Zusammensetzung zur Verwendung oder Verwendung nach wenigstens einem der vorhergehenden Ansprüche, wobei die Zusammensetzung 29 Gew.-% Flavonoide und 19 Gew.-% Polyphenole umfasst.

**9.** Zusammensetzung zur Verwendung oder Verwendung nach wenigstens einem der vorhergehenden Ansprüche, wobei die Zusammensetzung 22 Gew.-% Mangiferin, 4 Gew.-% Catechin, 3 Gew.-% Epicatechin und 0,4 Gew.-% Quercitindihydrat umfasst.

**10.** Zusammensetzung zur Verwendung oder Verwendung nach wenigstens einem der vorhergehenden Ansprüche, wobei die Zusammensetzung in dem Subjekt Superoxid-Dismutase inhibiert.

**11.** Zusammensetzung zur Verwendung oder Verwendung nach wenigstens einem der vorhergehenden Ansprüche, wobei die Zusammensetzung ein Pulver, eine Pille, eine Tablette, ein Pellet, eine Kapsel, ein dünner Film, eine Lösung, ein Spray, ein Sirup, ein Hustensaft, eine Lutschtablette, eine Pastille, ein Kaugummi, eine Paste, ein Dampf, eine Suspension, eine Emulsion, eine Salbe, eine Creme, eine Lotion, ein Einreibemittel, ein Gel, ein Tropfen, ein topisches Pflaster, ein bukkales Pflaster, eine Kugel, ein Naschgummi oder eine Injektion ist.

**Revendications**

**1.** Composition comprenant un extrait de *Mangifera* pour une utilisation dans le traitement de l'obésité, **caractérisée en ce que** l'extrait est un extrait d'écorce, de feuilles et de peaux des fruits de *Mangifera indica.*

**2.** Utilisation non thérapeutique d'une composition comprenant un extrait de *Mangifera* dans la gestion du poids chez un sujet, **caractérisée en ce que** l'extrait de *Mangifera* est un extrait d'écorce, de feuilles et de peaux des fruits de *Mangifera indica*.

**3.** Composition pour une utilisation selon la revendication 1, ou utilisation selon la revendication 2, **caractérisée en ce que** l'extrait de *Mangifera* est présent selon une quantité de 500 mg.

**4.** Composition pour une utilisation, ou utilisation, selon au moins l'une des revendications précédentes, pour la modification d'au moins un parmi l'appétit, le gain de poids corporel, le métabolisme ou l'absorption de calories.

**5.** Composition pour une utilisation, ou utilisation, selon au moins l'une des revendications précédentes, pour la modulation d'au moins un parmi les taux d'insuline, les taux de lipase et la peroxydation des lipides.

**6.** Composition pour une utilisation, ou utilisation, selon au moins l'une des revendications précédentes, pour la modulation d'au moins un parmi les taux de glucose sérique, de cholestérol total, de triglycérides, de HDL-c et de LDL-c.

**7.** Composition pour une utilisation, ou utilisation, selon au moins l'une des revendications précédentes, **caractérisée en ce que** l'extrait d'écorce, de feuilles et de peaux des fruits de *Mangifera indica* est un extrait d'écorce, de feuilles et de peaux des fruits de *Mangifera indica* selon un rapport de 80:10:10, respectivement.

**8.** Composition pour une utilisation, ou utilisation, selon au moins l'une des revendications précédentes, **caractérisée en ce que** la composition comprend, en poids, 29% de flavonoïdes et 19% de polyphénols.

9.  Composition pour une utilisation, ou utilisation, selon au moins l'une des revendications précédentes, **caractérisée en ce que** la composition comprend, en poids, 22% de mangiférine, 4% de catéchine, 3% d'épicatéchine, et 0,4% de dihydrate de quercétine.

10. Composition pour une utilisation, ou utilisation, selon au moins l'une des revendications précédentes, **caractérisée en ce que** la composition inhibe la superoxyde dismutase chez le sujet.

11. Composition pour une utilisation, ou utilisation, selon au moins l'une des revendications précédentes, **caractérisée en ce que** la composition est une poudre, une pilule, un comprimé, un granulé, une capsule, un film fin, une solution, un spray, un sirop, un looch, une tablette, une pastille, un chewing-gum, une pâte, une vapeur, une suspension, une émulsion, un onguent, une crème, une lotion, un liniment, un gel, des gouttes, un patch topique, un patch buccal, des billes, une gomme ou une injection.

Fig. 1a

Fig 1b

Fig. 1c

Fig 1d

Fig. 1e

Fig. 1f

FIG. 2

**Fig. 3a**

**Fig 3b**

Fig. 3c

**Total Anti-oxidant assay**

Fig 3d

**Anti-lipase assay**

Fig. 3e

Fig. 3f

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- OECD Guideline for the testing of chemicals- Repeated Dose 90-day Oral Toxicity Study in Rodents. *TG 408,* 21 September 1998
- Acute Oral Toxicity'' -Fixed Dose Procedure. *OECD Guideline No. 420,* 17 December 2001
- **CHOUGULE A. ; MANJUNATH A.** Anti-obesity drugs of Bhava prakasha nighantu: The literary survey. *IJRAP,* 2012, vol. 3 (5), 650-654
- **JULIANA APARECIDA SEVERI ; ZEILA PINHEIRO LIMA ; HÉLIO KUSHIMA ; ALBA REGINA MONTEIRO SOUZA BRITO ; LOURDES CAMPANER DOS SANTOS ; WAGNER VILEGAS ; CLÉLIA AKIKO HIRUMALIMA.** Polyphenols with Antiulcerogenic Action from Aqueous Decoction of Mango Leaves (Mangifera indica L.). *Molecules,* 2009, vol. 14, 1098-1110
- **TAING M.W. ; PIERSON J-T ; HOANG V.L. T. ; SHAW P. N. ; DIETZGEN R. G. ; GIDLEY M. J. ; SARAH J. ; THOMSON R ; MONTEITH G. R.** Mango fruit peel and flesh extracts affect adipogenesis in 3T3-L1 cells. *Food Funct.,* 2012, vol. 3, 828-836
- **MORENO, D. A. ; RIPOLL, C. ; ILIĆ, N. ; POULEV, A. ; AUBIN, C. ; RASKIN, I.** Inhibition of lipid metabolic enzymes using Mangifera indica extracts. *J. Food Agric. & Environ.,* 2005, vol. 4 (1), 21-26
- **AJILA, C. M. ; BHAT, S. G. ; PRASADA RAO, U. J. S.** Valuable components of raw and ripe peels from two Indian mango varieties. *Food Chemistry,* 2007, vol. 102, 1006-1011
- **AIYELAAGBE O.O. ; OSAMUDIAMEN P. M.** Phytochemical Screening for Active Compounds in Mangifera indica Leaves from Ibadan. *Oyo State Plant Sciences Research,* 2009, vol. 2 (1), 11-13
- **SHAH K.A. ; PATEL M.B. ; PATEL R.J. ; PARMAR P.K.** Mangifera Indica (Mango). *Phcog. Rev.,* 2010, vol. 4 (7), 42-48
- **WAUTHOZ N. ; BALDE A. BALDE E. S. ; DAMME M. V. ; DUEZ P.** Ethnopharmacology of Mangifera indica L. Bark and Pharmacological Studies of its Main C-Glucosylxanthone, Mangiferin. *International Journal of Biomedical and Pharmaceutical Sciences,* 2007, vol. 1 (2), 112-119
- **SINGH S. K. ; SINHA S. K. ; PRASAD S.K. ; KUMAR R. ; BITHU B. S. ; KUMA S. S. ; SINGH P.** Synthesis and evaluation of novel analogues of mangiferin as potent antipyretic. *Asian Pacific Journal of Tropical Medicine,* 2011, 866-869
- **GABINO GARRIDO ; IDANIA RODEIRO ; IVONES HERNÁNDEZ ; GASTÓN GARCIA ; GEMA PÉREZ ; NELSON MERINO ; ALBERTO NÚÑEZ-SELLÉS ; RENÉ DELGADO.** *In vivo acute toxicological studies of an antioxidant extract from Mangifera indica L. (Vimang),* 2009, vol. 32 (1), 53-8
- **FUCHUAN GUO ; CONGHUI HUANG ; XILU LIAO ; YEMEI WANG ; YING HE ; RENNAN FENG ; YING LI ; CHANGHAO SUN.** Beneficial effects of mangiferin on hyperlipidemia in high-fat-fed hamsters. *Molecular Nutrition & Food Research,* 2011, vol. 55 (12), 1809-18